# EUROPEAN PATENT APPLICATION

(11) **EP 1 595 956 A1**
(43) Date of publication of application: **16.11.2005**
(21) Application number: 04011388.8
(22) Date of filing: 13.05.2004
(51) Int. Cl.: C12P 1/00, C12P 7/00, C12N 9/02

(54) **Process for an enzymatic oxygenation by direct electrochemical regeneration of the FAD-dependent monooxygenase**

(71) Applicant: BASF Aktiengesellschaft, 67056 Ludwigshafen (DE)
(72) Inventor: Schmid, Andreas, Dr., 8049 Zürich (CH); Hollmann, Frank, 8046 Zürich (CH); Hofstetter, Karin, 8093 Zürich (CH); Habicher, Tilo, Dr., 67346 Speyer (DE); Hauer, Bernhard, Prof.Dr., 67136 Fussgönheim (DE)

(57) **Abstract**

Process for an enzymatic oxygenation catalyzed by a FAD-dependant monooxygenase and direct electrochemical regeneration of the FAD-dependant monooxygenase

## Description

### Background

Selective oxyfunctionalization of unreactive hydrocarbons still represents one of the most challenging frontiers of synthetic organic chemistry. Especially the delicate balance of reactant-activation and selectivity of the reaction has to be dealt with. 'Classical' chemical oxygen donors such as peroxides, hypochlorites, iodosobenzenes, or dioxiranes ^{[1]} lack the selectivity which is required for oxyfunctionalizations of more complex substrates.

Furthermore, most catalytic chemical approaches are not very far developed yet, so that turnover numbers and frequencies as well as the stereodiscrimination of the catalysts tend to be low. ^{[2, 3]} Nature on the other hand has developed a versatile toolbox of catalysts meeting exactly the aforementioned criteria:

Monooxygenases catalyze highly diversified oxygenation reactions generally in a very regio- and stereoselective manner at catalyst performances reaching several hundred turnovers per minute. ^{[4]} The reactive oxygenating species is generated *in situ* from molecular oxygen at the monooxygenase's active site thereby minimizing undesired side reactions. Thus, monooxygenases are promising catalysts to be used in synthetic organic chemistry. ^{[5-8]} In return however, monooxygenases are cofactor-dependent enzymes, which have to be supplied with reducing equivalents for O₂ activation. Generally those reducing equivalents are derived from the costly and instable nicotinamide cofactors (NAD(P)H). ^{[9-11]} Furthermore, monooxygenases often are composed of complex multienzyme systems accomplishing the electron transfer from NAD(P)H to the terminal oxygenase. Due to the sophisticated molecular architecture and the NAD(P)H dependency, preparative applications of monooxygenases - with few exceptions - ^{[8, 12-16]} have been largely confined to whole-cell approaches using metabolically active microorganisms. ^{[5, 8, 17-19]}

Given the complexities of mimicking the native monooxygenase cycle, direct introduction of reducing power into the oxygenation cycle offers the possibility of drastic simplification biocatalytic oxyfunctionalization reactions. Electrochemical reduction is one approach of choice since the reducing power applied can be controlled and the cathode serves as reagent-free source of electrons. In this respect, the class of hemedependent monooxygenases so far has been the favored subject of research. Electrical communication between the monooxygenase's heme-iron center and the cathode was established either by direct contact, ^{[20, 21]} and via artificial ^{[22]} or biological redox relays ^{[23-25]} mediating the electron transfer.

In contrast to the varied research activities on P450 monooxygenases, similar approaches for the class of flavin-dependent monooxygenases have not been reported yet, which is astonishing insofar, as this enzyme class catalyzes synthetically interesting oxyfunctionalization reactions such as hydroxylations, ^{[12, 26, 27]} Baeyer-Villiger oxidations, ^{[28, 29]} and epoxidations. ^{[30]}

Styrene monooxygenase (StyAB) from *Pseudomonas sp.* VLB120 catalyzes the specific *(S)*-epoxidation of a broad range of styrene derivatives. ^{[31, 32]} The enzyme is composed of a FAD-dependent monooxygenase component (StyA) that catalyzes the epoxidation reaction and a NADH-dependent reductase component (StyB) delivering the reducing equivalents from NADH to StyA via FADH₂. ^{[33]}

Previously, we have shown that StyB is not directly involved in the epoxidation reaction since it can be replaced by chemical reductants without impairment of the stereochemical course or the rate of the reaction. ^{[34]} There, *in situ* regeneration of FADH₂ was achieved using the organometallic complex [Cp*Rh(bpy)(H₂O)]²⁺ as transfer hydrogenation catalyst together with formate as stochiometric source of reducing equivalents.

### Description of the invention

The following invention relates to a process for an enzymatic oxygenation of an educt E to a product P catalyzed by an FAD-dependant monooxygenase, characterized in that the FAD-dependant monooxygenase is regenerated by direct electrochemical reduction.

The chemical nature of the educt E can be varied in a broad range as long as an monooxygenase, especially an FAD-dependant monooxygenase is able to accept the educt E as a substrate for oxygenation. Preferred as educt E are compounds substituted styrenes and styrene derivatives, especially preferred are the subtrates mentioned in table 1.

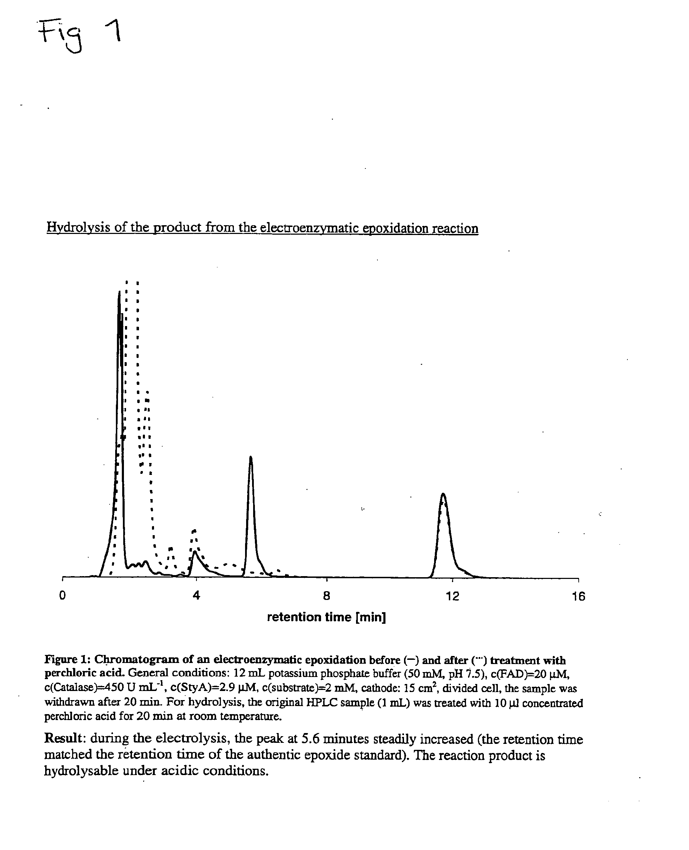

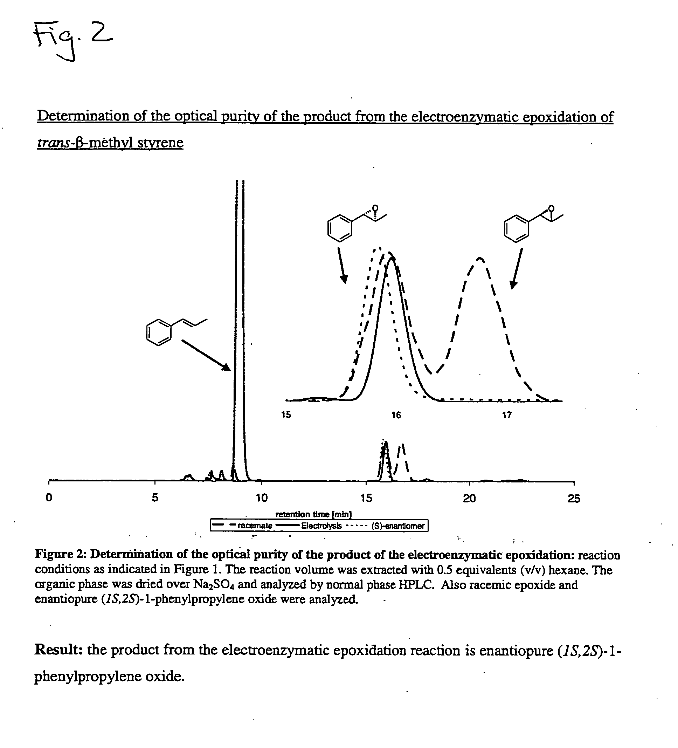

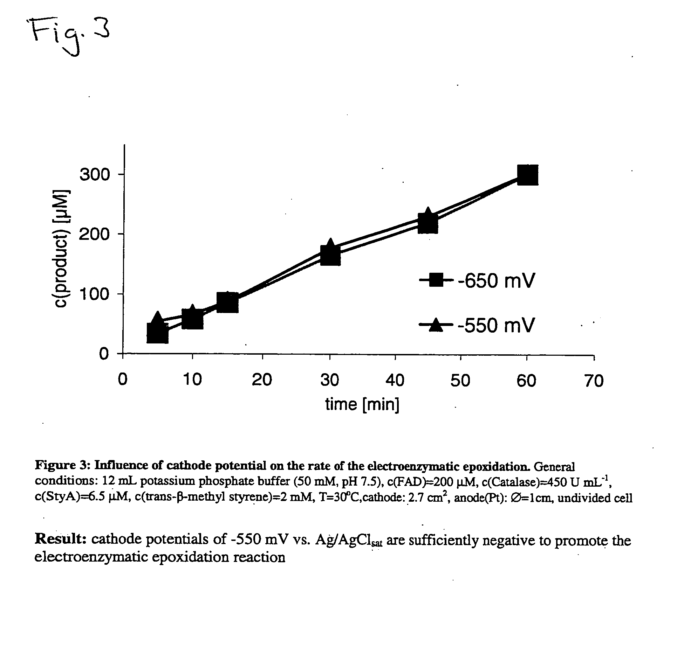

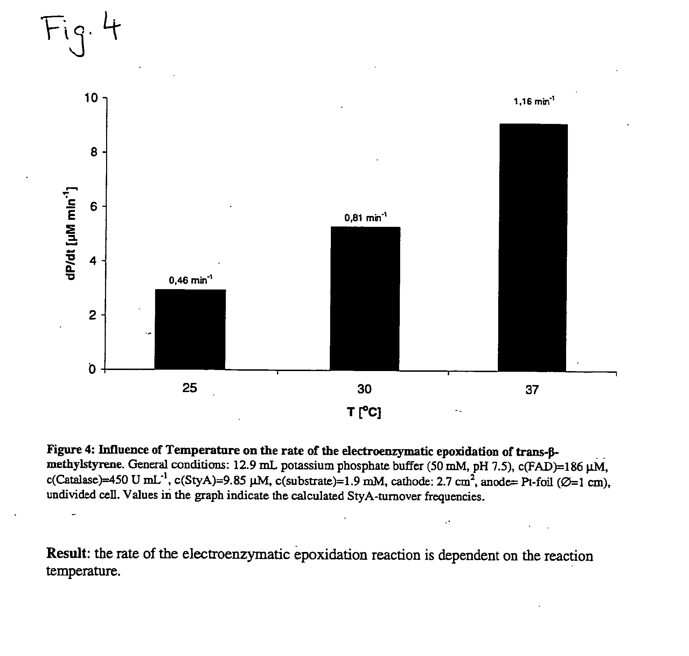

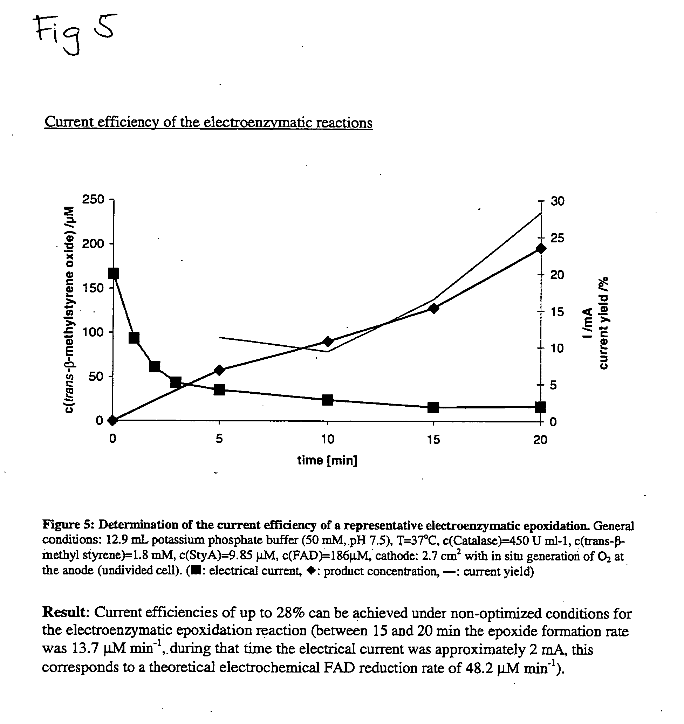

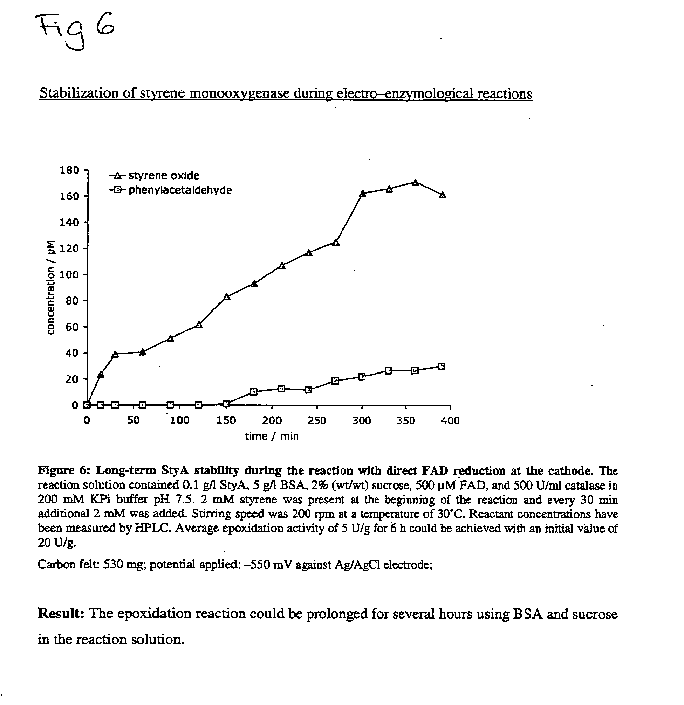

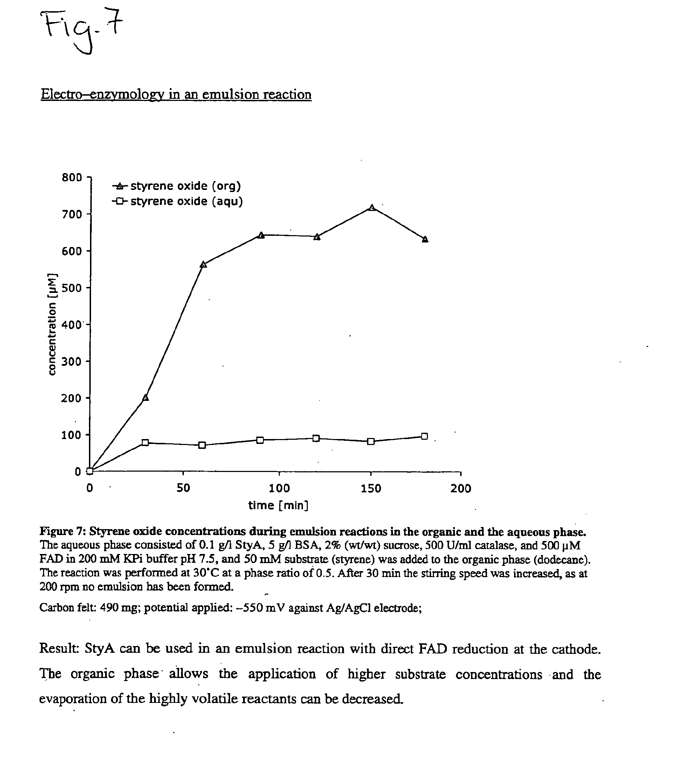

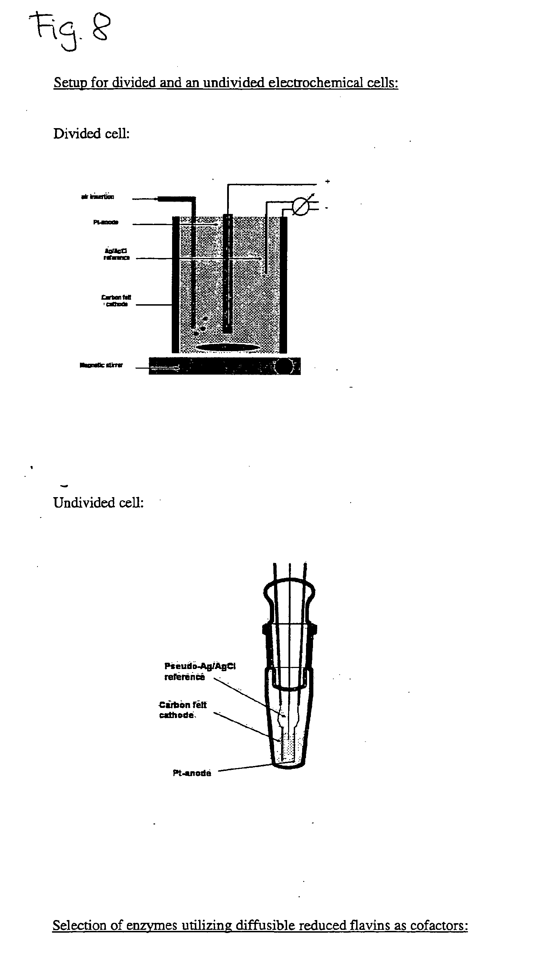

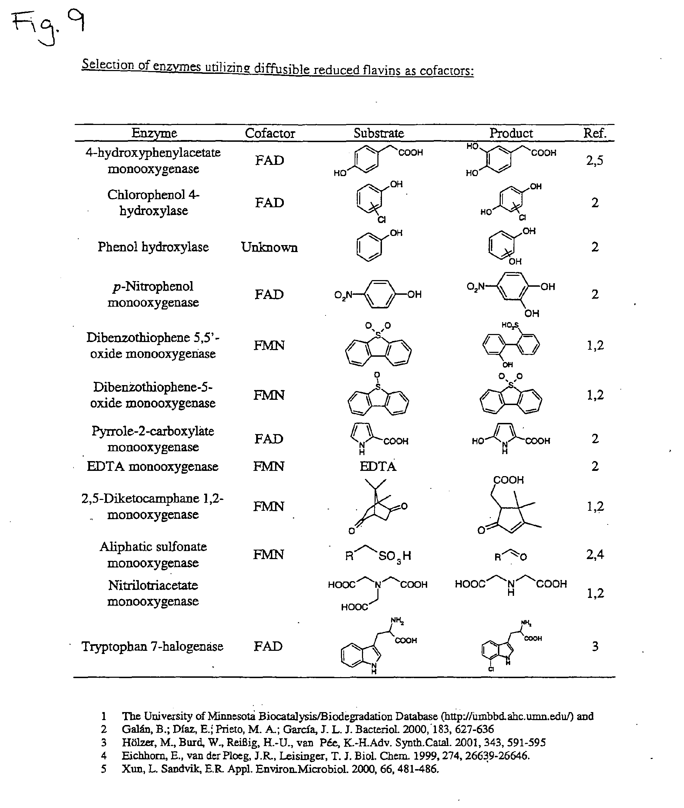

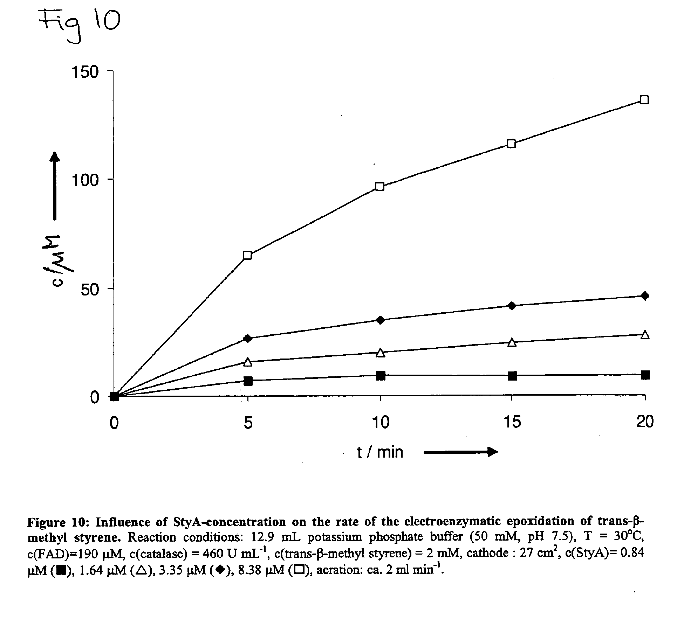

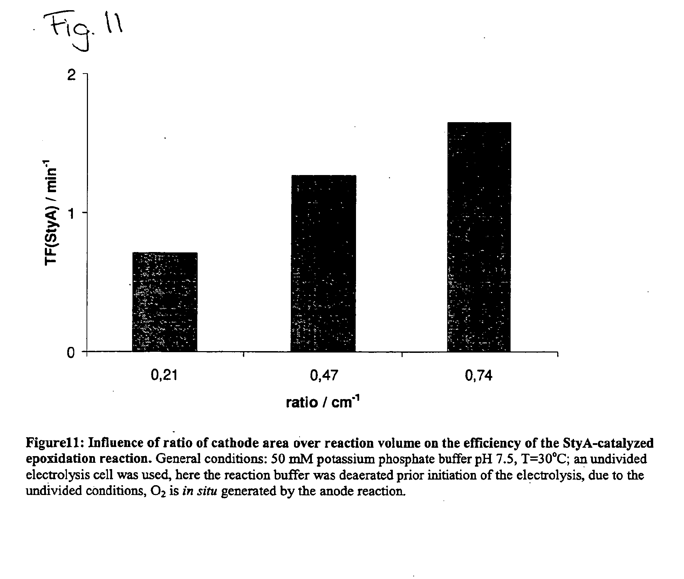

As monooxygenase according to the invention are preferred the styrene monoooxygenase (Sty AB) from Pseudomonas sp. ^{[31, 32]} Other preferred enzymes are listed in Fig. 9.

Initial experiments on the electroenzymatic epoxidation were performed with *trans-β*-methyl styrene as substrate. Electrolyses were performed potentiostatically applying a cathode potential of -550 mV vs. Ag/AgCl_{sat.}. No product formation was detectable when either StyA or FAD was omitted from the reaction medium. On the other hand, electrolyses in the presence of all reaction components yielded the formation of a hydrolysable, more polar product, which was confirmed to be practically enantiopure (*1S,2S*)-1-phenylpropylene oxide. ^{[36]} Similarly, a broad variety of diversely substituted vinylaromatic compounds could be transformed to the more than 98% optically pure corresponding *(S)* epoxides (Table 1).

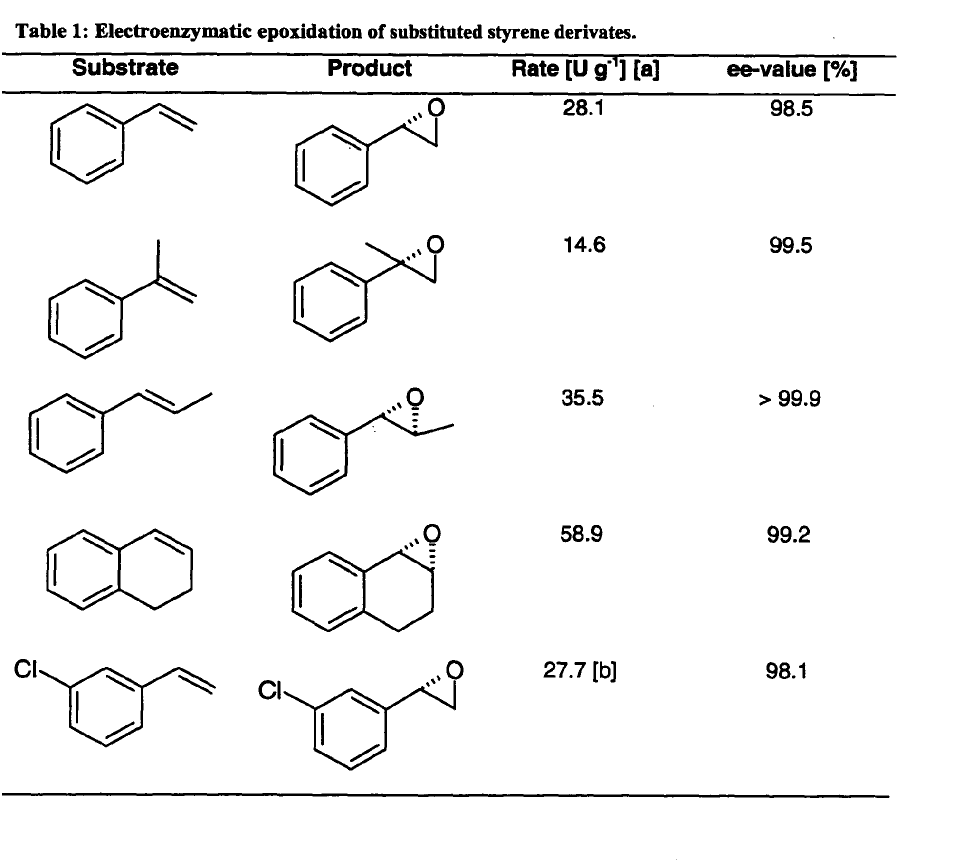

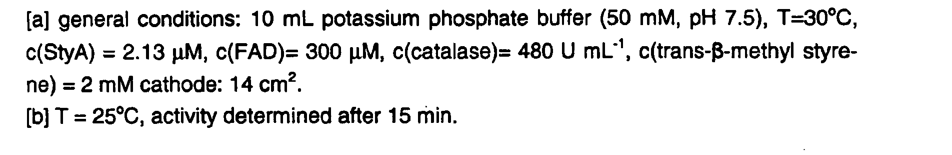

However, while the stereodiscrimination of the electroenzymatic oxygenation reactions met the values obtained with whole-cells ^{[31, 32]} as well as cell-free reactions ^{[34, 35]}, the epoxidation rate was comparably poor. In initial-rate studies, specific StyA-activities up to 2.1 U mg⁻¹ had been determined. ^{[33]} Thus, the rates depicted in Table 1 constitute only a fraction (less than 2%) of the catalytic potential of StyA. With the goal of determining the rate-limiting factors of the presented electroenzymatic epoxidation reaction, we further investigated the influence of varying reaction parameters on the rate of the electroenzymatic epoxidation reaction.

As shown in **Fehler! Verweisquelle konnte nicht gefunden werden.0,** the rate of the electroenzymatic epoxidation reaction correlated with the biocatalyst concentration applied. Specific StyA activities of 35.5 ± 2.1 U g⁻¹ were observed independent from the biocatalyst concentration. This specific activity was temperature-dependent as increasing of the reaction temperature from e.g. 25°C to 37°C resulted in a 2.5-fold increase of epoxidation activity under otherwise identical conditions. ^{[36]} Thus, at a first glance, StyA appeared to be rate-limiting in the electroenzymatic reaction. However, the poor catalytic performance of StyA compared to maximal values suggested yet other factors severely limiting the rate of the electroenzymatic epoxidation reaction.

Lowering the cathode potential from -550 to -650 mV vs. Ag/AgClₛₐₜ did not significantly influence the reaction course ^{[36]} suggesting that the electron transfer from the cathode to FAD was not rate-limiting for the regeneration of FADH₂. As heterogeneous reaction, however, the regeneration of FADH₂ may be limited by mass transport to the cathode surface. In fact, we observed that increasing FAD-concentrations up to at least 500 µM resulted in increasing epoxidation rates. ^{[36]} These results are contrary to previous findings where a defined optimal FAD concentration between 10 and 20 µM was observed using homogeneous regeneration of FADH₂. ^{[33, 34]} There, autocatalytic oxidation of FADH₂^{[37]} accounted for the decrease of epoxidation rate at FAD concentrations higher than 20 µM. In the present case, this effect may be overruled by the increased FADH₂ generation rate due to the increased availability of FAD at the cathode surface. Provided the latter assumption was correct and cathodic FADH₂ regeneration is subject to diffusion limitation, also the cathode surface should affect the regeneration rate. Therefore, the influence of ratio of cathode surface to reaction volume was investigated. As shown in 11, the specific StyA activity (here depicted as turnover frequency [catalytic cycles per minute]) correlated directly with the ratio of cathode areas and reaction volume.

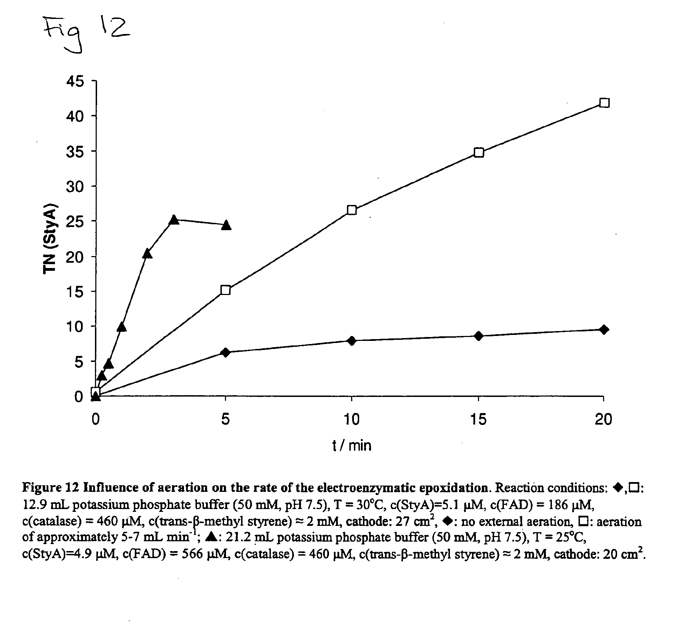

Altogether, these observations suggested that StyA activity in the electroenzymatic epoxidation reaction is limited by the availability of FADH₂ for the epoxidation reactions. Since reduced flavins are not stable in the presence of molecular oxygen, ^{[37]} we investigated the influence of aeration on the rate of the electroenzymatic epoxidation reaction (Figure 12).

Interestingly, we found that increasing aeration rates drastically accelerated the epoxide formation rate. Without active intake of air a specific StyA activity was in the range of 30 U g⁻¹ was determined reaction (Figure 12). Furthermore, only approximately 50 µM of epoxide were overall formed, suggesting that more than 80% of the dissolved oxygen is consumed by reactions other than the enzymatic epoxidation. High aeration rates on the other hand increased the specific StyA activity up to 215 U g⁻¹ corresponding to approximately 10% of the maximal StyA activity. This is interesting since studies on the direct reductive regeneration of P450 monooxygenases identified oxidative uncoupling of the electrochemical regeneration reaction from the enzymatic oxygenation reaction to be overall limiting. ^{[22, 24, 38]} For example, Vilker and coworkers found a drastic increase in P450_{cam}-driven hydroxylation of camphor if the electrolysis buffer was Ar-purged prior applying the cathode potential and in situ-regeneration of O₂ at the anode. This apparent discrepancy may be explained considering the mechanism of FADH₂ oxidation ^{[37]} as outlined in Scheme 1.

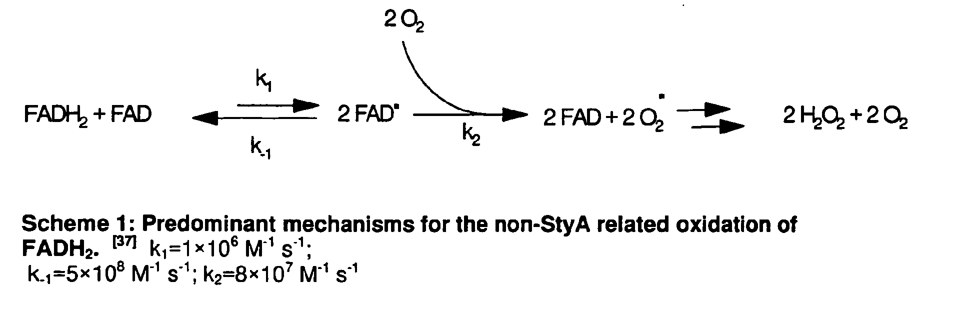

Accordingly, the formation of the semiquinone radical anion by reversible synproportionation limits the overall rate for the non-enzyme-supported oxidation of reduced flavins. Thus, in our experiments c(O₂) did not influence the rate of the non-enzyme supported re-oxidation of FADH₂. On the other hand, molecular oxygen is involved directly in the formation of the catalytically active 4α-peroxoflavin. Provided this is the overall rate-limiting step of the StyA-catalyzed epoxidation reaction, this would sufficiently explain the dependence of the epoxidation rate on the aeration rate. The latter assumption is supported by similar findings with the FAD-dependent *p*-hydroxyphenylacetate-3-hydroxylase where formation of 4α-peroxoflavin was found to be rate-limiting and O₂-dependent. ^{[39]} Future experiments will examine the influence of the *in situ* concentration of O₂ on the electroenzymatic reaction more deliberately.

One particular challenge for the preparative application of the new electroenzymatic epoxidation reaction so far is its comparably low long-term stability. Generally, the reactions ceased after 1 to 1.5 h. From the results obtained so far, some qualitative conclusions can be drawn. First, a correlation of the overall reaction time with the total protein content applied can be detected (compare also **Fehler! Verweisquelle konnte nicht gefunden werden.0)** and, second, the reaction times decrease with the rate of air intake **(Fehler! Verweisquelle konnte nicht gefunden werden.12)** Both observations point towards a low stability of the biocatalyst under the reaction conditions. This low stability of StyA may partially be due to the absorption of StyA to the cathode surface were it is exposed to locally high concentrations of partially reduced oxygen originating from cathodic reduction of O₂. ^{[40]} Furthermore, the heterogeneous intake of O₂ brings about the occurrence of shear forces and surface tensions at the liquid-gaseous interface destabilizing the three-dimensional structure of the biocatalyst. Previous studies suggested a beneficial influence of additional 'sacrificial' proteins such as bovine serum albumin (BSA) ^{[35]} also heterogenzation of StyA, e.g. via immobilization to Eupergit C may be viable. Further studies aiming towards increased biocatalyst stability under the conditions are underway.

In conclusion, our study demonstrates for the first time the direct electrochemical regeneration of a flavin-dependent monooxygenase. Driven only by electrical power, optically pure epoxides were synthesized from corresponding vinyl aromatic compounds. Thus, the rather complicated native electron transport chain consisting of 3 polypeptides (StyA, StyB, and a NADH regenerating enzyme) and 2 cofactors (NADH and FAD) could be cut down to the components absolutely necessary for the epoxidation a maximally simple biocatalytic epoxidation reaction. Now, having shown the usefulness of the electroenzymatic approach to simplify such complicated enzyme system it may be extended to other enzymatic oxygenation reactions ^{[41]} making synthetically interesting reactions such as oxidative desulphurization ^{[42]}, specific hydroxylation of aromatic rings ^{[43-45]}, enantioselective Baeyer-Villiger Reactions, ^{[46]} and even selective halogenation reactions ^{[47]} feasible using only the isolated monooxygenases and FAD in an electrochemical cell.

### Experimental Section

Chemicals were purchased from Fluka (Buchs, Switzerland) in the highest purity available and used without further purification.
StyA was enriched from recombinant *Escherichia coli* JM101 as described previously ^{[35]}. The purity of the lyophilized biocatalyst was approximately 70% (as determined by SDS gel-electrophoresis).

Electrolyses were performed in a thermostatted stirred tank reactor. Cylindrical carbon felt served as cathode (working electrode) and the potential was adjusted versus a saturated Ag/AgCl_{sat.} reference electrode. The dimensions of the working electrode are given a macroscopic area (corresponding to an average of 27.1 ± 2.1 mg cm⁻²). Conditions of either a divided or an undivided cell were chosen. For the divided cell, the Pt-wire counter electrode was placed in a dialysis membrane; otherwise a Pt-foil (∅1cm) was used. After supplementing the reactor with the reaction components indicated a cathode potential of -550 mV vs. Ag/AgCl_{sat.} was applied. In case of divided cell, O₂ was supplied by heterogeneous intake of air (intake rates were estimated with a Hewlett Packard soap film flowmeter; under the conditions of an undivided cell, O₂ was generated at the counter electrode.

Reaction rates (and enzyme performances calculated thereof) were determined based on the product formation as determined by HPLC using protocols previously reported. ^{[34, 35]}

### References

[1] W. Adam, W. Malisch, K. J. Roschmann, C. R. Saha-Moller, W. A. Schenk, *Journal of Organometallic Chemistry* **2002,** *661, 3.*
[2] J.-M. Brégeault, *Dalton Transactions* **2003,** 3289.
[3] B. Comils, W. A. Herrmann, *Applied Homogeneous Catalysis with Organometallic Compounds,* Wiley-VCH, Weinheim, **2002.**
[4] R. B. Silverman, *The organic chemistry of enzyme-catalyzed reactions,* Academic Press, San Diego, **2002.**
[5] Z. Li, J. B. van Beilen, W. A. Duetz, A. Schmid, A. de Raadt, H. Griengl, B. Witholt, *Current Opinion in Chemical Biology* **2002**, *6*, 136.
[6] S. G. Burton, *Trends in Biotechnology* **2003**, *21*, 543.
[7] H. E. Schoemaker, D. Mink, M. G. Wubbolts, *Science* **2003,** *299*, 1694.
[8] A. Schmid, J. S. Dordlick, B. Hauer, A. Kiener, M. Wubbolts, B. Witholt, *Nature* **2001,** *409*, 258.
[9] H. K. Chenault, G. M. Whitesides, *Applied Biochemistry and Biotechnology* **1987,** *14*, 147.
[10] K.-H. Drauz, H. Waldmann, *Enzyme catalysis in organic synthesis,* 2 ed., Wiley-VCH, Weinheim, **2002.**
[11] W. A. van der Donk, H. Zhao, *Current Opinion in Biotechnology* **2003,** *14*, 421.
[12] J. Lutz, V. V. Mozhaev, Y. L. Khmelnitsky, B. Witholt, A. Schmid, *Journal of Molecular Catalysis B: Enzymatic* **2002,** *19-20*, 177.
[13] S. C. Maurer, H. Schulze, R. D. Schmid, U. Urlacher, *Advanced Synthesis and Catalysis* **2003,** *345,* 802.
[14] U. Schwarz-Linek, A. Krödel, F.-A. Ludwig, A. Schulze, S. Rissom, U. Kragl, V. I. Tishkov, M. Vogel, *Synthesis* **2001,** *6*, 947.
[15] F. Zambianchi, P. Pasta, G. Carrea, S. Colonna, N. Gaggero, J. M. Woodley, *Biotechnology and Bioengineering* **2002**, *78*, 489.
[16] S. Rissom, U. Schwarz-Linek, M. Vogel, V. I. Tishkov, U. Kragl, *Tetrahedron: Asymmetry* **1997,** *8*, 2523.
[17] A. J. J. Straathof, S. Panke, A. Schmid, *Current Opinion in Biotechnology* **2002,** *13*, 548.
[18] A. Liese, M. Villela Filho, *Current Opinion in Biotechnology* **1999,** *10*, 595.
[19] C. Wandrey, A. Liese, D. Kihumbu, *Organic Process Research and Development* **2000,** *4*, 286.
[20] J. Kazlauskaite, A. C. G. Westlake, L.-L. Wong, H. A. O. Hill, *Chemical Communication* **1996,** 2189.
[21 ] C. Lei, U. Wollenberger, C. Jung, F. W. Scheller, *Biochemical and Biophysical Research Communications* **2000,** *268,* 740.
[22] K. M. Faulkner, M. S. Shet, C. W. Fisher, R. W. Estabrook, *Proceedings of the National Academy of Science of the United States of America* **1995,** *92,* 7705.
[23] M. P. Mayhew, V. Reipa, M. J. Holden, V. L. Vilker, *Biotechnology Progress* **2000,** *16,* 610.
[24] V. Reipa, M. P. Mayhew, V. L. Vilker, *Proceedings of the National Academy of Science of the United States of America* **1997**, *94*, 13554.
[25] V. L. Vilker, V. Reipa, M. P. Mayhew, M. J. Holden, *Journal of the American Oil Chemists' Society* **1999,** *76*, 1283.
[26] A. Schmid, I. Vereyken, M. Held, B. Witholt, *Joumal of Molecular Catalysis B: Enzymatic* **2001,** *11,* 455.
[27] M. J. H. Moonen, M. W. Fraaije, I. M. C. M. Rietjens, C. Laane, W. J. H. van Berkel, *Advanced Synthesis and Catalysis* **2002,** *344*, 1023.
[28] V. Alphand, G. Carrea, R. Wohlgemuth, R. Furstoss, J. M. Woodley, *Trends in Biotechnology* **2003***, 21,* 318.
[29] M. D. Mihovilovic, B. Muller, P. Stanetty, *European Journal of Organic Chemistry* **2002,** *22,* 3711.
[30] S. Colonna, N. Gaggero, G. Carrea, G. Ottolina, P. Pasta, F. Zambianchi, *Tetrahedron Letters* **2002,** *43,* 1797.
[31] S. Panke, B. Witholt, A. Schmid, M. G. Wubbolts, *Applied and Environmental Microbiology* **1998,** *64,* 2032.
[32] A. Schmid, K. Hofstetter, H.-J. Feiten, F. Hollmann, B. Witholt, *Advanced Synthesis and Catalysis* **2001,** *343,* 732.
[33] K. Otto, K. Hofstetter, M. Rötlisberger, B. Withott, A. Schmid, *submitted* **2004.**
[34] F. Hollmann, P.-C. Lin, B. Witholt, A. Schmid, *Journal of the American Chemical Society* **2003**, *125, 8209.*
[35] K. Hofstetter, J. Lutz, I. Lang, B. Witholt, A. Schmid, *Angewandte Chemie International Edition in English* **2004,** *43,* 2163.
[36] See supplementing information.
[37] V. Massey, *The Journal of Biological Chemistry* **1994**, *269*, 22459.
[38] U. Schwaneberg, D. Appel, J. Schmitt, R. D. Schmid, *Journal of Biotechnology* **2000,** *84,* 249.
[39] U. Arunachalam, V. Massey, S. Miller, *Journal of Biological Chemistry* **1994,** *269*, 150.
[40] F. Hollmann, A. Schmid, E. Steckhan, *Angewandte Chemie International Edition in English* **2001,** *40*, 169.
[41] B. Galán, E. Díaz, M. A. Prieto, J. L. Garcia, *Joumal of Bacteriology* **2000***, 183,* 627.
[42] E. Eichhorn, J. R. van der Ploeg, T. Leisinger, J. *Biol. Chem.* **1999**, *274*, 26639.
[43] M. R. Gisi, L. Xun, J. *Bacteriol.* **2003,** *185*, 2786.
[44] P. Chaiyen, C. Suadee, P. Wilairat, *Eur J Biochem* **2001**, *268,* 5550.
[45] D. Becker, T. Schrader, J. Andreesen, *Eur J Biochem* **1997**, *249*, 739.
[46] D. G. Taylor, P. W. Trudgill, *Journal of Bacteriology* **1986,** *165*, 489.
[47] S. Keller, T. Wage, K. Hohaus, M. Hölzer, E. Eichhorn, K.-H. van Pée, *Angewandte Chemie International Edition in English* **2000,** *39,* 2300.

## Claims

1. Process for an enzymatic oxygenation of an educt E to a product P catalyzed by an FAD-dependant monooxygenase, **characterized in that** the FAD-dependant monooxygenase is regenerated by direct electrochemical reduction.

2. Process according to claim 1 where the oxygenation reaction is an epoxidation.

3. Process according to claim 1 where the oxygenation reaction is an oxidative desulphurization.

4. Process according to claim 1 where the oxygenation reaction is an enantioselective Baeyer-Villiger reaction.

5. Process according to claim 1 where the oxygenation reaction is a hydroxylation of an aromatic molecule.

6. Process according to claim 1 where the FAD-dependant monooxygenase is 4-hydroxyphenylacetate-monooxygenase

7. Process according to claim 1 where the FAD-dependant monooxygenase is pyrrole-2-carboxylate-monooxygenase.

8. Process according to claim 1 where the FAD-dependant monooxygenase is chlorophenol-4-hydroxylase.

9. Process according to claim 1 Where the educt E is a substituted or unsubstituted styrene.

10. Process according to claim 1 where the FAD-dependant monooxygenase is the styrene monooxygenase (Sty AB) from pseudomonos.
